# EUROPEAN PATENT APPLICATION

(11) **EP 4 431 007 A1**
(43) Date of publication of application: **18.09.2024**
(21) Application number: 22892267.0
(22) Date of filing: 12.12.2022
(51) Int. Cl.: A61B 5/00, A61B 5/372, H04R 1/10

(54) **SLEEP MONITORING DEVICE AND OPERATION METHOD THEREFOR**

(30) Priority: 12.11.2021 KR 20210155975; 24.05.2022 KR 20220063458; 14.07.2022 KR 20220087045
(71) Applicant: Slowave Inc., Seoul 06160 (KR)
(72) Inventor: RYU, Kyung Ho, Anyang-si Gyeonggi-do 13973 (KR); RYU, Won Dae, Dangjin-si Chungcheongnam-do 31721 (KR)
(74) Representative: DREISS Patentanwälte PartG mbB
(86) International application number: PCT/IB2022/062049
(87) International publication number: WO 2023/084502

(57) **Abstract**

A user sleep monitoring device comprises: a communication interface; and a processor. The processor determines whether the user's brain waves correspond to sleeping brain waves or waking brain waves by using EEG signals measured by an EEG measurement unit of an earbud device worn on the user's ears, and classifies the user's sleep state into one of a plurality of categories by comparing the result of the determination with sensor data measured by an activity sensor included in the earbud device, wherein the sensor data is time-synchronized with the EEG signals.

## Description

### TECHNICAL FIELD

The following description relates to a medical device measuring biometric information, a healthcare device, and a wearable electronic device, and more particularly, a device for monitoring sleep.

### BACKGROUND ART

It may be known that sleep significantly affects the health and life quality of a human. The amount and quality of sleep may be important. Falling asleep well (entering sleep), sleeping well, and waking up well (exiting sleep) may affect sleep quality. The sleep quality of humans may be monitored by a brain wave identified by measuring an electroencephalogram (EEG) and body motion during sleeping may be monitored by an activity sensor. In addition, a sleep disorder, such as snoring or sleep apnea, may be monitored by a microphone to listen to snoring while sleeping and a measurement sensor for blood oxygen saturation (SpO2)
In a mental health care center of a large hospital or a clinic specialized in sleeping, polysomnography may be performed to monitor a sleep state and a sleep disorder event of a patient. However, polysomnography, such as measuring an electrocardiogram (ECG) by attaching multiple electrodes to the forehead, may cause inconvenience to the patient and may have a limitation that the monitoring is a one-time event only for the day of examination.

A biometric signal measurement device at a healthcare level that measures a brain wave by wearing the device on the forehead in the form of band in daily life, rather than at a special clinic, has been introduced, and an application for recording a sleep time in a smartwatch has been introduced. However, in an aspect of sleep monitoring, a technique that allows the user to monitor their sleep quality by themselves has not been generalized due to the limitation, such as inconvenience of measurement and inaccuracy of sleep state determination.

The Korean Patent No. 10-2046515 (publication date: November 29, 2019) provides a healthcare system using a neckband-type EEG measurement device.

The United States Patent No. US2016/0058366 (publication date: March 3, 2016) provides a sleep monitoring method in a watch-type wearable device worn on a wrist.

The United States Patent No. US2019/0038184 (publication date: February 7, 2019) provides a watch-type wearable device for tracking a user's activity.

The Korean Patent No. 10-2102421 (publication date: April 20, 2020) provides earphones for brain wave measurement and a drowsy driving detection system using thereof.

### DISCLOSURE OF THE INVENTION

### TECHNICAL GOALS

When monitoring a sleep state, whether a posture of a user is a lie-down posture may be accurately recognized. A sleep state of a user may be more accurately and continuously measured by measuring and comparing the posture of the user, a motion, and/or shaking of the body together with a brain wave (or electroencephalogram (EEG)).

The continuity and persistency of all-night sleep monitoring may be secured by maintaining wearing as an electrical contact between user's skin and a signal electrode for measuring the brain wave of the user is stably maintained.

When the user has a sleep disorder in entering sleep or during sleeping, a guide for entering sleep or sleeping stages may contribute to sleep monitoring as well as improvement of sleep quality.

Monitoring the sleep state and sleep disorder of the user may help them wake up refreshed by appropriately inducing sleep stages when waking up (when exiting sleep). The device may contribute to improvement of the sleep quality of the user.

### TECHNICAL SOLUTIONS

### Categorizing a sleep state by considering an activity sensor with EEG

According to an aspect, a sleep monitoring device is provided. The sleep monitoring device may include a communication interface and a processor. According to one embodiment, the processor may identify whether a brain wave of a user corresponds to a sleep brain wave (or a "sleep wave") or an awake brain wave (or an "awake wave") using an EEG signal measured by an EEG measurement unit of an earbud device worn on the user's ear. In addition, the processor may classify a sleep state of the user as one of a plurality of categories by comparing sensor data (or data from an activity sensor) measured by an activity sensor included in the earbud device with the identified result, wherein the sensor data is time synchronized to the EEG signal.

According to one embodiment, the processor may identify whether the sensor data measured by the activity sensor corresponds to an activity posture or a lie-down posture and may classify (or categorize) the sleep state of the user as one of a plurality of states including 1) lie-down posture and sleep wave state (SS), 2) activity posture and awake wave state (AA), 3) lie-down posture and awake wave state (SA), and 4) activity posture and sleep wave state (AS).

For example, but without being limited thereto, the processor may continuously classify the sleep state of the user as one of the plurality of states during a predetermined time interval between a first time point corresponding to entering sleep of the user and a second time point corresponding to exiting sleep of the user. In addition, the processor may provide a result sequence of the state classification over time as a sleep monitoring result for the user. The result sequence of the state classification may be, for example, a time sequential arrangement array of state categories classified in the flow of sleep time or may be a visualized graph thereof.

According to one embodiment, the processor may classify the sleep state of the user as one of the plurality of states at a predetermined cycle during the time interval. For example, but without being limited thereto, the predetermined cycle may be a time unit, such as 30 seconds, one minute, five minutes, or 10 minutes. According to one embodiment, the result sequence of the state classification may be an arrangement of classification results every 5 or 10 minutes (or may be a different time unit), wherein the classification results are obtained by classifying the sleep state of the user from the time of falling asleep to the time of waking up as one of 1) lie-down posture and sleep wave state (SS), 2) activity posture and awake wave state (AA), 3) lie-down posture and awake wave state (SA), and 4) activity state and sleep wave state (AS).

### Improving assessment accuracy by removing or reducing outliers

Meanwhile, since the sleep state of the user is continuously determined during a relatively long sleep time, for example, a long time of about 7 hours, the processor may improve the stability of the determination result by removing an outlier or noise of a temporary signal. Various embodiments are possible for the process.

According to one embodiment, when the sleep state of the user is classified as one of the plurality of states at the predetermined cycle, the processor may determine the sleep state of the user to be one of the plurality of states at each cycle to determine the sleep state while applying a smoothing filter to at least one of the EEG signal in one cycle and the sensor data measured by the activity sensor to remove an outlier. When the smoothing filter is applied as described above, an outlier of a temporary signal or a noise impact may be removed or reduced.

According to another embodiment, a removal of an outlier may be performed by determining a sleep state at a sub-period time segment that segments an individual time interval into a smaller unit and then may determine a sleep state that is dominant, in other words, more frequently appears, in the entire individual time interval, to be a representative state of the entire individual time interval. Naturally, when determining the sleep state in the entire individual time interval or a time segment, which is a sub-period time segment, the processor may determine the sleep state using the EEG signal and the sensor data measured by the activity sensor.

According to another embodiment, the removal of the outlier in an individual time interval or a sub-period time segment may be processed using temporal locality. For example, an unusual state for determining that the sleep state of the user is not actually changed may be ignored by referring to one or more processing results that are previous or subsequent. In addition, the removal of the outlier may be possible in various methods.

### Providing a sleep quality assessment result

Meanwhile, after the sleep monitoring process is terminated, the sleep quality monitoring device may perform a sleep quality assessment for a total monitored time interval, for example, from light-off in which a user lies in bed to light-on in which the user wakes up. According to one embodiment, the processor may provide a sleep quality index corresponding to a ratio of a sum of intervals classified as the lie-down posture and sleep wave state (SS) to a total time interval, based on the classification result.

In this case, the total time interval subjected to sleep monitoring may begin, for example, when the user changes their state from the activity posture and awake wave state (AA) to the lie-down posture and awake wave state (SA). As another example, the total time interval may begin when the "SA" state continues for more than a predetermined period (or number of times). In the case of exiting sleep, the total time interval subjected to sleep monitoring may be determined when the user changes their state from the SS state in which the user lies down and sleeps to the SA state in which the user is awakened from sleep but does not get up, and the total time interval may also be determined when the " SA" state continues for more than a predetermined period (or number of times).

In addition, a first change from the "SA" state to the "SS" state in which the user lies down but does not fall asleep and then finally falls asleep may be determined to be a non-rapid eye movement (REM) sleep level 2, which is not REM sleep, from sleep levels determined by a sleep monitoring device 101 by analyzing an EEG signal of an earbud device 100. During sleep, REM sleep and non-REM level 1 sleep may be determined to be the "SS" state, but a first entry to sleep may be determined to be a start time of a non-REM level 2 stage in which the user sleeps at a predetermined depth.

For example, but without being limited thereto, a ratio of a sum SSn, which is a sum of time lengths of the lie-down posture and sleep wave state (SS), to a total sleep time T may be assessed as the total sleep quality (or "sleep efficiency", hereinafter, the "sleep quality" shall be construed as a concept that includes the "sleep efficiency" throughout the present disclosure). Since a state in which the user lies down to sleep and then raises their body (if the user is awake, the state is AA, and if not, the state is AS) and a state in which the user lies down but is awake (SA) are not good for the overall sleep efficiency, when such states do not appear or are short, the sleep quality index may increase.

### Estimating and analyzing sleep disorder or physical disease (suspected) states other than sleep quality

In addition, the sleep quality may be variously determined. Accordingly, a sleep habit of the user may be analyzed by assessing whether a cause that decreases the sleep quality index is the "AA" state, the "AS" state, or the "SA" state. For example, a sleep disorder action, such as raising the body during sleep or even walking, may be identified if the "AS" state, in which the brain wave is the sleep wave but the user raises their body, continues for a predetermined time or more. In addition, when the "SA" state, in which the user is awake but still lies down, appears a predetermined number of times or more during the total assessment time, a sleep disorder related to awakening during sleep may be suspected by analyzing in which pattern the state appear, how long the state continues, or when the "SA" state occurs, and in regard to this, the user may have a consultation with an expert, such as a doctor.

When the "AA" state in which the user shows an activity posture and an awake wave occurs during the total sleep assessment period, termination of sleep may be determined depending on whether the state continues and the assessment may be terminated. When the state is equal to or shorter than a preset time and changes to the sleep wave state ("AS" or "SS), the sleep assessment may be continued, the sleep may be considered as one sleep, and the occurrence of the "AA" state may be included in the assessment. The case described above may appear when the user is awake for a moment and goes to a bathroom and if the case is repeated, it may be used as a ground for suspecting a sleep disorder or a urinary disease.

### Additional care for increasing the assessment accuracy

Meanwhile, the activity sensor may include multiple individual sensors, such as a gyro sensor, an acceleration sensor, or a composite sensor including two or more sensor fusions. According to one embodiment, the sensor data measured by the activity sensor may include a) a direction sensing value that determines whether the user erects or lays their head while wearing the earbud device on their ears, and b) an acceleration sensing value that determines whether the user moves at a predetermined level or more while wearing the earbud device on their ears.

In a case in which the user sleeps with another person rather than sleeping alone, an unexpected event may occur by various causes from the other person as well as a cause from the user and in this case, the classification of an expected sleep state may be hindered. For example, a sensing value may not accurately reflect a sleep state of the user because the body of a person sleeping next to the user contacts or hits the user. In addition, various circumstances may be possible.

Accordingly, according to one embodiment, the processor may temporarily defer the sleep state classification when the acceleration sensing value indicates the state in which the user moves at the predetermined level or more.

In addition, according to one embodiment, the earbud device may provide a microphone listening result for detecting snoring during the sleep of the user, and in this case, the assessment of when, how much, how long, and how severely the user snores may be additionally performed and if needed, recording may be performed, and in this case, the snoring of the user and snoring of the person sleeping next to the user may need to be distinguished. Accordingly, the processor in one embodiment may further receive a sound collected through the microphone of the earbud device and when the sound corresponds to snoring, the processor may distinguish whether the sound is snoring of the user or other surrounding person using the acceleration sensing value.

For example, but without being limited thereto, when vibration at a predetermined level is sensed together with the snoring sound, the sound may be the snoring of the user and when the snoring sound is collected but the vibration is less than or equal to the predetermined level, the sound may be the snoring of the person sleeping next to the user, and thereby, this may be ignored in the sleep state assessment of the user, who wears the earbud device and sleeps, subjected to monitoring.

### EFFECTS OF THE INVENTION

According to embodiments, a sleep disorder, such as sleep apnea, is measured together with a brain wave (an EEG) of a user by measuring blood oxygen saturation (SpO2) and a sleep state of the user is more accurately and continuously measured by measuring and comparing whether a posture of the user is a lie-down posture with the brain wave (the EEG).

According to a device in various embodiments, as an electrical contact between a signal electrode used for EEG measurement and user's skin is stably maintained, a success rate of sleep monitoring during sleep (all-night) increases. As the EEG is implemented in a single device with user activity sensing, the accuracy and persistency of sleep posture monitoring increases.

Since continuous monitoring and accurate data are stably secured during sleep, time sequential statistics may be provided and a precise analysis and utilization in various fields may be allowed. When a user has a sleep disorder in entering sleep or during sleeping, precise utilization, such as when and which type of sleep disorder does the user have, may be possible.

In addition, a more relevant sleep monitoring may be performed by removing an outlier and various detailed assessments may be performed by comparing various sensing values. For example, an event during a sleep state of the user as described above may be distinguished from an event of a person sleeping next to the user.

Furthermore, for providing user experience (UX), since a sleep monitoring device is able to provide a voice and/or image guide for entering sleep and inducing sleep stages through an earbud device, the sleep monitoring device may monitor sleep and provide a result as well as may directly contribute to improvement of sleep quality of the user.

As described above, the sleep monitoring device not only performs state monitoring when entering sleep and during sleep but also induces sleep stages appropriately when the user wakes up from sleep (exiting sleep) to help the user wake up refreshed.

### BRIEF DESCRIPTION OF DRAWINGS

FIG. 1 illustrates a sleep monitoring device according to one embodiment.
FIGS. 2A and 2B are front view and rear view of an earbud device according to one embodiment, respectively.
FIG. 3 is a diagram illustrating a process of sleep monitoring while wearing an earbud device and sleeping according to one embodiment.
FIG. 4 is a diagram illustrating an earbud device including a forehead expansion electrode according to another embodiment and FIG. 5 illustrates a user wearing the device and sleep monitoring is performed.
FIG. 6 illustrates a process of classifying a sleep state according to one embodiment.
FIG. 7 illustrates a reason that an earbud-type device has an advantage over a conventional watch-type device for sleep monitoring.
FIG. 8 illustrates an example of a result screen in which sleep states are time sequentially generated and visualized according to one embodiment.
FIG. 9 is an example of a screen suggesting an example of a sleep disorder and sleep quality in a sleep monitoring result according to one embodiment.
FIG. 10 is a diagram illustrating a sensor used by a sleep monitoring device to monitor various sleep disorder events together with an earbud device according to one embodiment.

### BEST MODE FOR CARRYING OUT THE INVENTION

Hereinafter, the examples will be described in detail with reference to the accompanying drawings. The scope of the right, however, should not be construed as limited to the embodiments set forth herein. In the drawings, like reference numerals are used for like elements.

The terms used herein are selected from terms generally understood by those skilled in the related art, but may have different meanings according to technical developments and/or changes, practices, and preferences of an engineer. Accordingly, the terms used herein should not be construed as limiting the technical spirit, and should be construed as illustrative terms to describe embodiments.

In addition, in a specific case, most appropriate terms are arbitrarily selected by the applicant. In this instance, the meanings of the arbitrarily used terms will be clearly explained in the corresponding description. Hence, the terms should be understood not by the simple names of the terms but by the meanings of the terms and the following overall description of this specification.

A sleep monitoring device 101 according to one embodiment may be an electronic device. The electronic device may include all types of system-on-chip hardware and/or a general-purpose computing device implemented by application software if the device performs a sleep monitoring function.

For example, the electronic device may include at least one of a smartphone, a tablet personal computer (PC), a mobile phone, a video phone, an e-book reader, a desktop PC, a laptop PC, a netbook computer, a personal digital assistant (PDA), a portable multimedia player (PMP), an MP3 player, a mobile medical device, a camera, or a wearable device (e.g., a head-mounted-device (HMD) such as electronic glasses, an electronic garment, an electronic bracelet, an electronic necklace, an electronic appcessory, an electronic tattoo, or a smartwatch).

According to an additional embodiment, the electronic device may include at least one of various medical devices (e.g., magnetic resonance angiography (MRA), magnetic resonance imaging (MRI), computed tomography (CT), an imaging device, an ultrasound device, etc.), a navigation device, a global positioning system (GPS) receiver, an event data recorder (EDR), a flight data recorder (FDR), a vehicle infotainment device, an electronic device for vessel (e.g., a marine navigation system and a gyro compass, etc.), avionics, a security device, a vehicle head unit, an industrial or household robot, an automatic teller's machine (ATM) of a financial institution, or a point of sales (POS) of a store.

In addition, the electronic device may include at least one of a portion of a building, a structure, or furniture including a sleep monitoring function, an electronic board, an electronic signature receiving device, a projector, or various measuring instruments (e.g., water, electricity, gas, or radio wave measuring instruments, etc.). The electronic device according to various embodiments of the present disclosure may be one of the various devices described above or a combination thereof. In addition, the electronic device according to various embodiments of the present disclosure may be a flexible device. It is obvious to one having ordinary skill in the art that the type of the electronic device may be various and the type and main purpose (other than the sleep monitoring function) of the electronic device are not limited to the descriptions provided above. Hereinafter, detailed descriptions of embodiments are provided with reference to drawings.

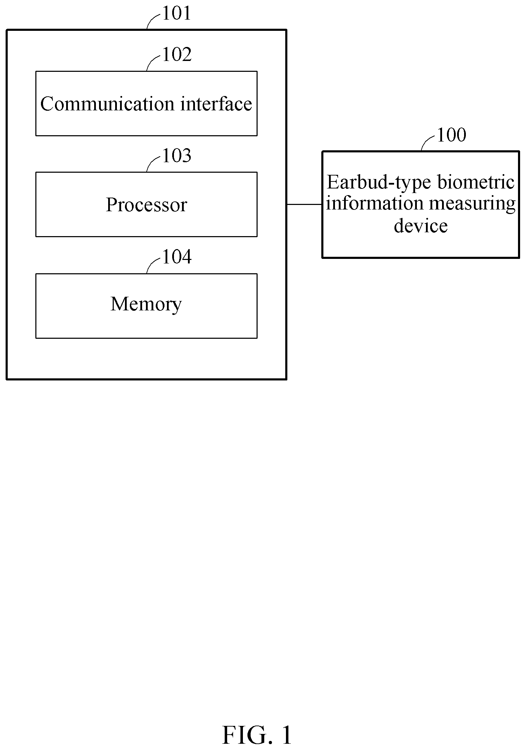

FIG. 1 illustrates the sleep monitoring device 101 according to one embodiment.

### Connection between the sleep monitoring device 101 and an earbud device 100

According to one embodiment, the sleep monitoring device 101 may be the various types of electronic device described above and may be connected to the earbud-type biometric information measuring device 100 via a communication interface 102 to receive sensed data and exchange a control command. In this case, the earbud device 100 may be worn as an in-ear type on an ear of the user and the sleep monitoring device 101 may be separately carried by the user. Various communication schemes via the communication interface 102 may be possible, such as Bluetooth, ZigBee, Wi-Fi, Wi-Fi direct, connection via a cellular network, and near field communication (NFC).

However, the example is not limited thereto and in another embodiment, the sleep monitoring device 101 may be embedded in the earbud device 100. Due to the high integration and high performance of system-on-chip, a processor 103 and a memory 104 may be packaged in the earbud device 100 worn on the user's ear, and in this case, the communication interface 102 may transmit a monitoring result of the sleep monitoring device 101 to another electronic device (not shown) and the user may check the sleep monitoring result through the another electronic device, as necessary. Accordingly, even if the embodiment in which the sleep monitoring device 101 and the earbud device 100 are separately provided devices is provided, it shall not be construed as being limited to the example.

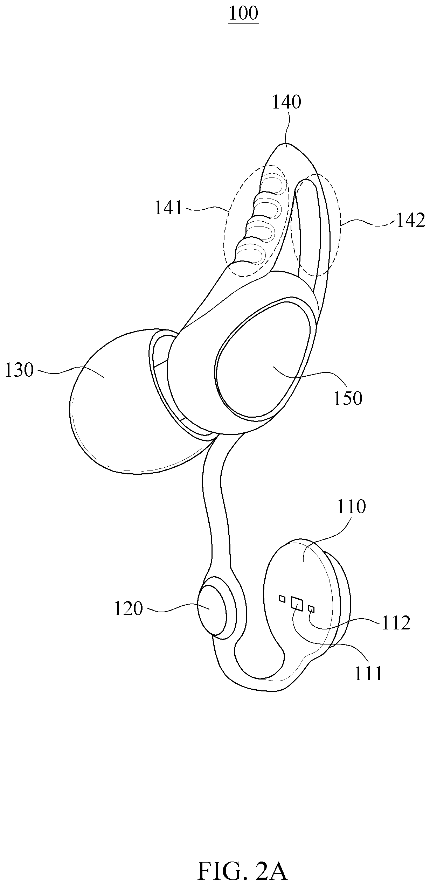

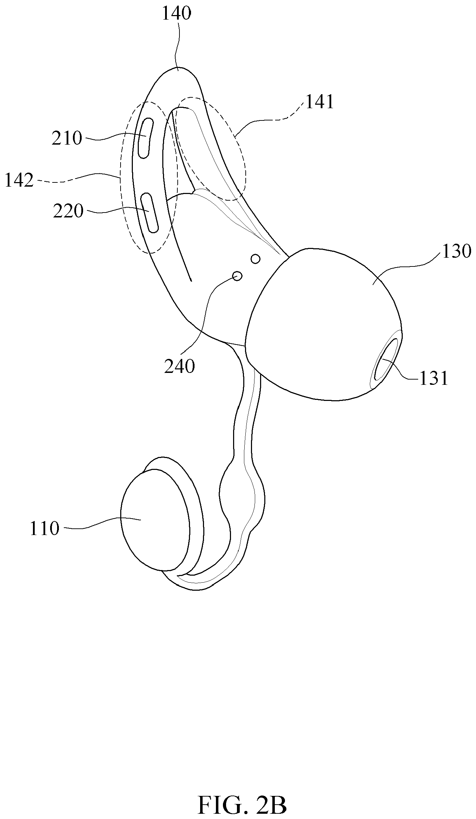

A structure and a function of the earbud device 100 are further described with reference to FIGS. 2A and 2B and hereinafter, a structure and an operation of the sleep monitoring device 101 are described.

### Configuration of the sleep monitoring device 101

According to one embodiment, the sleep monitoring device 101 may include the communication interface 102 and the processor 103. The communication interface 102 may be a communication chipset or a modem that enables various types of the communications described above. Preferably, it may be a wireless communication chipset. The processor 103 may be not only system-on-chip (SoC) dedicated hardware but also a general-purpose processor, such as a central processing unit (CPU) and an auxiliary processing unit. An application processing unit (APU) used for a smartphone or a smartwatch may also be possible. In the case of the general-purpose processor, operations to be described below may be performed by firmware, an operating system (OS), and application software (APP), and the application software may be installed using the memory 104. The description is similar to a configuration and operations of a typical electronic device and is similar to the implementation of an electronic device, such as a wearable device known to one having ordinary skill in the art. Hereinafter, a characteristic and a difference of an operation of the sleep monitoring device 101 are described.

### Categorizing a sleep state by considering an activity sensor with EEG

According to one embodiment, the processor 103 may identify whether an electroencephalogram (EEG) of the user corresponds to a sleep brain wave (or a "sleep wave") or an awake brain wave (or an "awake wave") using an EEG signal measured by an EEG measurement unit of the earbud device 100 worn on the user's ear. The identification may be construed as the selection or estimation of one from two options. A type and a state of an EEG waveform of the user may be divided in a method known in an existing sleep neuroscience field depending on a waveform of alpha wave, beta wave, theta wave, and the like. Purportedly, a sleep state may be divided into rapid eye movement (REM), sleep levels 1 to 3, and the like, depending on the EEG waveform and the processor 103 may segment them, but in the present disclosure, the processor 103 may choose either the waveform corresponds to sleep or awakening. In addition, the processor 103 may choose either a posture of the user is a lie-down posture (or a sleep posture) or a state in which the user raises their head and is awake (or in an awake posture) through sensor data measured by an activity sensor included in the earbud device 100, wherein the sensor data is time synchronized to an EEG signal. The awake posture may refer to a posture in which the user sits or stands and may be clearly distinguished from a posture in which the user lies down with their head on the floor. As described above, the "activity sensor" herein may include various individual sensors, such as a gyro sensor, an acceleration sensor, or composite sensors including two or more sensor fusions, and may transmit a sensing value included in the earbud device 100 to the processor 103, and the processor 103 may distinguish whether the user raises their head or lays their head on the floor or a pillow through the sensing value.

Eventually, the EEG of the user may be determined to be one of two states (sleep wave vs. awake wave) and the posture of the user may be divided into one of two states (sleep posture vs. awake posture), and thereby, a total number of cases may be four. Depending on the embodiments, this may be further divided but in the embodiment described herein, the states may be four. The states are as follows:
1) lie-down posture and sleep wave state (SS: sleep posture and sleep wave);
2) activity posture and awake wave state (AA: awake posture and awake wave);
3) lie-down posture and awake wave state (sleep posture and awake wave); and
4) activity posture and sleep wave state (AS: awake posture and sleep wave).

The processor 103 may monitor a sleep state of the user in the flow of time through an EEG signal and measured by the earbud device 100 and an activity sensor signal and may continuously classify (categorize) the sleep state of the user as one of the four states described above in time series.

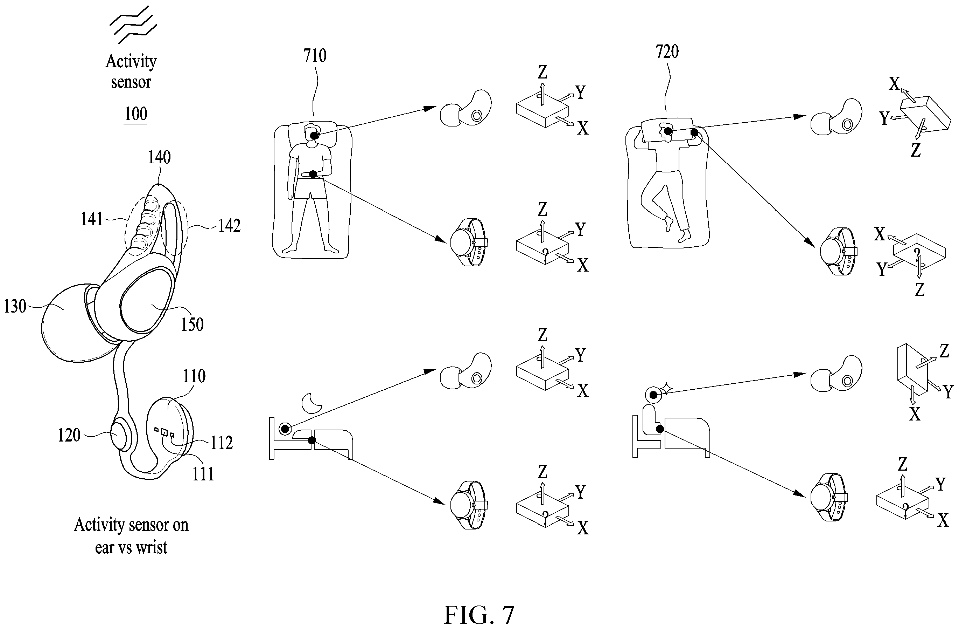

According to embodiments, the posture of the user may be considered together with the brain wave (EEG) of the user and since the earbud device 100 is inserted into the user's ear, the posture may be able to accurately distinguish the user's state by comparing whether the user actually lies down and falls asleep compared to checking the sleep state by a conventional watch-type wearable device. This will be further described with reference to FIG. 7.

Since the ears are on the head of the user, the earbud device 100 is inserted and worn on the ear, and the earbud device 100 in one embodiment stably maintains electrical contact between a signal electrode used for EEG measurement and the user's skin due to a structural feature of the earbud device 100, a success rate of sleep monitoring during the sleep (all-night) may increase, and thereby, accurate, reproducible, and stable sleep state monitoring may be possible according to embodiments.

### Time and cycle for monitoring the sleep state

For example, but without being limited thereto, the processor 103 may continuously classify the sleep state of the user as one of the plurality of states (SS, AA, SA, and AS) during a predetermined time interval between a first time point corresponding to an entry to sleep of the user and a second time point corresponding to exiting sleep of the user. A first time point, which is a time point of entering sleep, may be input by the user through a user interface (UI) (not shown) of the sleep monitoring device 101 and the device may automatically identify the first time point.

According to one embodiment, a case in which a sleep posture, which is a posture in which the user lies down to sleep, continues for a predetermined time or more, or a case in which a sleep wave having a predetermined length or more is firstly identified after the sleep posture continues may be determined to be the first time point.

The same applies to exiting sleep. The user may select and input a second time point, which is a time point of exiting sleep, through the UI on their will but according to one embodiment, the device may automatically identify this. For example, but without being limited thereto, the second time point, which is the time point of exiting sleep, may be determined when an awake wave of the user is sensed for a predetermined time or more while the sleep state of the user is monitored as the entry to sleep state is identified or even an awake posture is identified while the awake wave continues.

It may be difficult to observe in practice, but a theoretically ideal result may be a sequence in which the user is in the SA state (relatively short, for example, several minutes) as the user lies down to sleep, the SA continues, the user falls asleep while lying down and the SS state continues for a long time (for example, seven hours if the user sleeps for seven hours), the state changes to the SA state as the user wakes up from sleep while lying down, and then, the state changes to the AA state as the user raises their body within a relatively short time period and the AA state continues. The embodiments may allow the observation of how the ideal sequence is different from an actual sequence of the user. Since observation is required for analysis and the analysis is required for improvement or necessary treatment, the observation may be the beginning of improving the sleep quality.

According to one embodiment, the processor 103 may classify the sleep state of the user as one of the plurality of states (SS, AA, AS, and SA) at a predetermined cycle (e.g., every 30 seconds, 1 minute, 5 minutes, or 10 minutes) during the time interval.

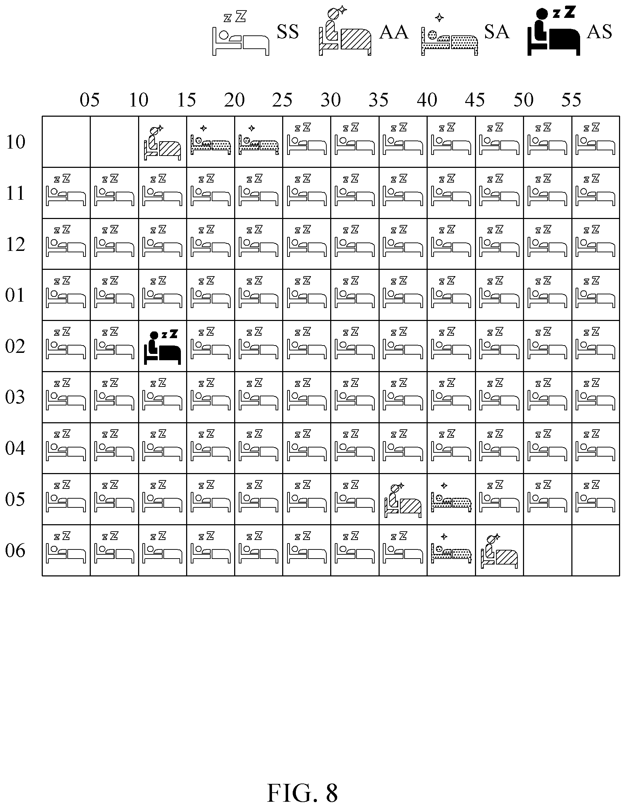

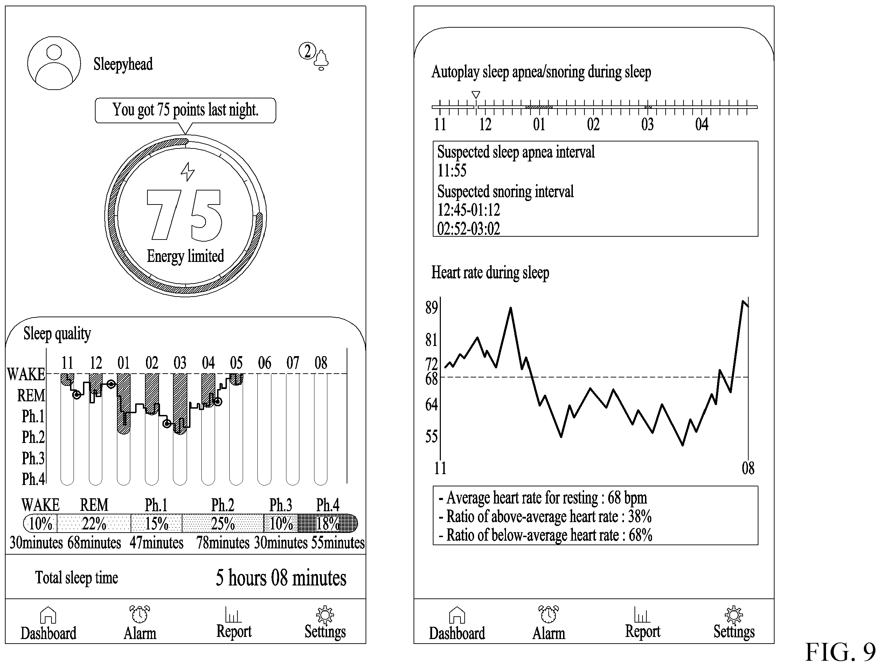

The classification result may be sequential visualized information or infographics in which the sleep states of the user are arranged by a determination cycle in the flow of time. The description thereof is further provided with reference to FIGS. 8 and 9.

### Processing of the initial state of entering sleep

Meanwhile, according to some embodiments, in a case of a first change from the "SS" state to the "SS" state, which is a time when the user lies down, fails to fall asleep, and then falls asleep, the processor 103 may consider a sleep level for determining the "SS" state. The timing of falling asleep may be differently set medically or depending on a field of information use. Accordingly, in some embodiments, while the processor 103 of the sleep monitoring device 101 analyzes the EEG signal of the earbud device 100 and determines the sleep level based on a waveform, the timing of entering a non-REM level 2 stage rather than REM sleep may be determined to be the "SS" stage in which the sleep begins. However, after the sleep begins based on the determination, the sleep level determined by the measured EEG may be determined to be the "SS" state regardless the sleep level is REM sleep or non-REM level 1. This is because a sleep state change between REM sleep and non-REM level 1 is natural depending on a cycle during the sleep.

### Improving assessment accuracy by removing or reducing outliers

Meanwhile, since the sleep state of the user is continuously determined during a relatively long sleep time, for example, a long time of about 7 hours, it may be important to remove an outlier or noise of a temporary signal by the processor 103. The stability and reliability of the determination result may be improved by removing the outlier. Various embodiments are possible for the process.

According to one embodiment, the processor 103 may apply a smoothing filter when classifying the sleep state of the user as the plurality of states at a predetermined cycle, for example, a cycle of 5 minutes or 10 minutes. For example, whether the user's brain wave is a sleep wave or an awake wave may be selected through an EEG signal of the user at a specific time interval, for example, 10 minutes, but an outlier may occur due to a cause, such as noise, and this may be removed by the smoothing filter, such as a temporal moving average filter.

In addition, a value of sensor data measured by the activity sensor in the specific time interval of 10 minutes may be continuously divided into a lie-down posture and an awaken posture, and an outlier may be removed from the value in a similar manner. Naturally, the removal of the outlier may be set at an optimal standard through various clinical experiments. Depending on the person, the user may raise their body and sit even if the user does not wake up from sleep and then may continue to sleep by lying down within a few seconds. The event described above may be the AS state appearing shortly for a few seconds during the SS state, and the processor 103 may not remove the event as an outlier and it may be important to properly identify and report the event as a sleep disorder action of the user.

Meanwhile, other embodiments are possible other than the smoothing filter. For example, a major state after segmentation may be determined to be a representative value. As if voting, when a plurality of different values exists in one interval, a value with the most count may be selected to be a representative value. In this embodiment, the processor 103 may segment one time interval, for example, 10 minutes, into a smaller sub-period time segment, for example, 1 minute, may determine a sleep state for each sub-period time segment, and then may determine a sleep state that is dominant, in other words, more frequently appears, in an entirety of the individual time interval (10 minutes), to be a representative state of the entire individual time interval. Naturally, when determining the sleep state in the entire individual time interval or a time segment, which is a sub-period time segment, the processor may determine the sleep state using the EEG signal and the sensor data measured by the activity sensor, may classify as one of SS, AA, SA, and AS, and may choose the representative value.

According to another embodiment, the removal of the outlier in an individual time interval or a sub-period time segment may be processed using temporal locality. For example, an unusual state for determining that the sleep state of the user is not actually changed may be ignored by referring to one or more processing results that is previous or subsequent. In addition, the removal of the outlier may be possible in various methods.

### Providing a sleep quality assessment result

Meanwhile, after the sleep monitoring process is terminated, the sleep monitoring device may perform a sleep quality assessment for the total monitored time interval. According to one embodiment, the processor may provide a sleep quality index corresponding to a ratio of a sum of intervals classified as the lie-down posture and sleep wave state (SS) to a total time interval, based on the classification result.

For example, but without being limited thereto, a ratio of a sum SSn, which is a sum of time lengths of the lie-down posture and sleep wave state (SS), to a total sleep time T may be assessed as the total sleep quality. Since a state in which the user lies down to sleep and then raises their body (if the user is awake, the state is AA and if not, the state is AS) and a state in which the user lies down but is awake (SA) are not good for the overall sleep efficiency, when such states do not appear or are short, the sleep quality index may increase. An example of the result is shown in FIG. 9.

### Estimating and analyzing sleep disorder or physical disease (suspected) states other than sleep quality

In addition, the sleep quality may be variously determined. Accordingly, a sleep habit of the user may be analyzed by assessing whether a cause that decreases the sleep quality index is the "AA" state, the "AS" state, or the "SA" state. For example, a sleep disorder action, such as raising the body during sleep or even walking, may be identified if the "AS" state, in which the brain wave is the sleep wave but the user raises their body, continues for a predetermined time or more. In addition, when the "SA" state, in which the user is awake but still lies down, appears a predetermined number of times or more during the total assessment time, a sleep disorder related to awakening during the sleep may be suspected by analyzing in which pattern the state appears, how long the state continues, or when the "SA" state occurs, and in regard to this, the user may have a consultation with an expert, such as a doctor.

When the "AA" state in which the user shows an activity posture and an awake wave occurs during the total sleep assessment period, termination of sleep may be determined depending on whether the state continues and the assessment may be terminated. When the state is equal to or shorter than a preset time and changes to the sleep wave state ("AS" or "SS), the sleep assessment may be continued, the sleep may be considered as one sleep, and the occurrence of the "AA" state may be included in the assessment. The case described above may appear when the user is awake for a moment and goes to a bathroom and if the case is repeated, it may be used as a ground for suspecting a sleep disorder or a urinary disease.

Furthermore, an appearance of "AS", which is a stage in which the user raises their body and sits or stands but the user is not awake while the "SS" in which the user lies down and sleeps repeats, this may be a serious sleep disorder even if the length of the appearance is short, and thereby, it may be separately classified as an event and may be reported to the user and/or a related service and may be notified.

Other various sleep disorders may occur but it may be unnecessary to list them in the disclosure and it shall be construed that the sleep monitoring device 101 and the earbud device 100 according to embodiments contribute to accurately and conveniently discovering various sleep disorders.

### Additional care for increasing the assessment accuracy

Meanwhile, the activity sensor may include multiple individual sensors, such as a gyro sensor, an acceleration sensor, or a composite sensor including two or more sensor fusions. According to one embodiment, the sensor data measured by the activity sensor may include a) a direction sensing value that determines whether the user erects or lays their head while wearing the earbud device 100 on their ears, and b) an acceleration sensing value that determines whether the user moves at a predetermined level or more while wearing the earbud device on their ears.

In a case in which the user sleeps with another person rather than sleeping alone, an unexpected event may occur by various causes from the other person as well as a cause from the user and in this case, the classification of an expected sleep state may be hindered. For example, a sensing value may not accurately reflect a sleep state of the user because the body of a person sleeping next to the user contacts or hits the user. In addition, various circumstances may be possible.

Accordingly, according to one embodiment, the processor may temporarily defer the sleep state classification when the acceleration sensing value indicates the state in which the user moves at the predetermined level or more.

In addition, according to one embodiment, the earbud device may provide a microphone listening result for detecting snoring during the sleep of the user, and in this case, the assessment of when, how much, how long, and how severely the user snores may be additionally performed and if needed, recording may be performed, and in this case, the snoring of the user and snoring of the person sleeping next to the user may need to be distinguished. Accordingly, the processor in one embodiment may further receive a sound collected through the microphone of the earbud device and when the sound corresponds to snoring, the processor may distinguish whether the sound is snoring of the user or other surrounding person using the acceleration sensing value.

For example, but without being limited thereto, when vibration at a predetermined level is sensed together with the snoring sound, the sound may be the snoring of the user and when the snoring sound is collected but the vibration is less than or equal to the predetermined level, the sound may be snoring of the person sleeping next to the user, and thereby, this may be ignored in the sleep state assessment of the user, who wears the earbud device and sleeps, subjected to monitoring.

FIGS. 2A and 2B are the front view and rear view of the earbud device 100 according to one embodiment, respectively.

### Example of configuration and front view of the earbud device 100

The earbud device 100 may be a wearable device worn on the user's ear. The earbud device 100 may measure biometric information, such as an EEG, a posture and a motion, and blood oxygen saturation (SpO2) of the user. When an EEG of a person is measured, a sleep stage, for example, REM sleep, sleep level 1, level 2, and level 3 may be distinguished. Information, for example, at which level the user falls asleep (a level when entering sleep) by identifying a dominant waveform, such as an alpha wave and a beta wave, by processing a brain wave signal, how a sleep stage changes during the sleep, what is a duration of each stage, and how many cycles taken for a change in the sleep waveform, may be monitored.

Furthermore, a sleep disorder during sleep, for example, sleep apnea, may be detected by measuring SpO2 and appropriate feedback may be provided. The feedback may be, for example, warning ears of a person through a sound, changing the sleep stage to another stage, or providing information on a sleep disorder through a connected application after the person wakes up from sleep.

Most conventional brain wave measuring devices are applied to a medical device level and at a healthcare device level that the user directly uses, a forehead electrode may be separately provided and may be inconvenient to wear. In some cases including the prior art provided in the present disclosure, the forehead electrode may be excluded to improve convenience, but it may be only an idea and may not be practical because when the user wears the brain wave measuring device during sleep, the continuity of measurement may not be maintained as an electrode may be removed from the user's skin (compared to a medical-level product). In addition, in the arrangement of electrodes, a reference electrode and signal electrodes may not be arranged in appropriate positions without interfering with each other.

According to one embodiment, the electronic device 100 may include a blood oxygen saturation measurement unit 110 that measures blood oxygen saturation SpO2 of the user while enclosing and being fixed to a lobule or lobe of the user, and a reference electrode that contacts the user's skin may be arranged therein. Since the reference electrode is in a position that less electrically interferes with an inside of an external auditory meatus and/or inner skin of an earflap on which an electrode is disposed, EEG measurement of the electronic device 100 may be excellent in terms of measurement accuracy, reproducibility, and convenience compared to conventional know products. A first part 110 of the blood oxygen saturation measurement unit may include a light-emitting diode (LED) light source 111 and a light receiver 112 and a second part 120 distinguished from the parts described above may include, for example but without being limited thereto, a fixing means, such as a magnet. With the earlobe in the middle, the first part 110 and the second part 120 may face each other and may be attached to each other by a magnetic force, the blood oxygen saturation measurement unit and the reference electrode for EEG measurement may be in contact with the earlobe. A portion between the first part 110 and the second part 120 may be a bridge formed of a flexible material. In addition, the drawing illustrates that the position of the light receiver 112 is at the first part 110, but the light receiver 112 may be placed on the second part 120.

Meanwhile, according to another embodiment, the bridge between the first part 110 and the second part 120 of the blood oxygen saturation measurement unit may be an elastic material having a U-shaped curve. In this case, the first part and the second part may be oriented to each other from respectively both sides of the earlobe by the elasticity of the elastic bridge with the earlobe therebetween and may hang on the earlobe. It may be designed to be fixed by being engaged with the earlobe.

According to another embodiment, since the connection bridge has a hinge structure, the first part 110 and the second part 120 may be folded and unfolded by the structure and may be fixed to the earlobe as the first part 110 and the second part 120 are open and closed. Other connection bridge structures may be possible and are not limited to the embodiments.

An ear tip 130 of the earbud device 100 may be inserted into a neck of an ear canal of the user and may have at least one in-ear electrode in contact with the skin in the ear canal. The ear tip 130 may be a part that outputs a sound, such as a typical in-ear type earbud earphone. Since the ear tip 130 is inserted into the neck of the ear canal and is closely in contact with the inner wall of the ear canal, the ear tip 130 may be an excellent signal electrode that maintains an electrically continuous electrical contact during the sleep of the user.

Meanwhile, the earbud device 100 may include an ear wing 140 that supports the ear tip 130 to push the ear tip 130 into the inside of the ear canal to prevent the ear tip 130 from being removed and is engaged and fixed to the inside of an antihelix of the user.

For example, but without being limited thereto, a wing structure of the ear wing 140 may have a loop shape. According to one embodiment, the wing structure may include an elastic structure 141 that is provided to correspond to various auricular sizes and structures of the user. The elastic structure 141 may include a plurality of slits and may have relatively great flexibility and/or elasticity compared to other parts. The great flexibility and/or elasticity may refer to having elasticity that the ear wing 140 is more deformed than other parts in the wing structure, for example, a portion 142 in which an ear wing electrode is disposed, by an external force, such as a condensation force or a twisting force, and when the external force weakens, the ear wing 140 is restored. The wing structure of the ear wing 140 may be selected from materials, such as elastomer, rubber, other silicone polymers, and the elastic structure 141 partially included in the wing structure may be processed or molded to be distinguished from other wing parts in terms of shape, but may include the same material. In addition, the material may be different. In this case, the elastic structure 141 may be deformed by the external force to cause the wing structure to be inserted into the antihelix and when the external force relatively weakens, the elastic structure 141 may restore the wing structure to be engaged with the inside of the antihelix and supported while causing at least one ear wing electrode is closely in contact with the contacting surface. Accordingly, the elastic structure 141 may help the ear wing electrode to be closely in contact with the inner skin of the antihelix of the user and therefore, may be advantageous to electrical contact for measuring an EEG signal. By the structure described above, a portion (142 of FIG. 2B) in which signal electrodes for EEG measurement are disposed may maintain electrical contact during the sleep of the user.

An EEG measurement unit 150 may collect an EEG signal of the user as the signal electrodes are disposed on the ear tip 130 and the portion 142 of the ear wing 140 and the reference electrode is disposed on the blood oxygen saturation measurement unit 110 that is in contact with the earlobe of the user. The EEG measurement unit 150 may include a circuit, such as a control unit, a flash memory, and various amplifiers, on a printed circuit board (PCB) or a flexible PCB (FPCB) and a communication chipset and an antenna for communicating with a sound driver that outputs a sound to the ear tip 130 and an external device (e.g., the sleep monitoring device 101 of FIG. 1) may be disposed in the EEG measurement unit 150.

According to one embodiment, the activity sensor may be included in a housing together with the EEG measurement unit 150. As described above, the activity sensor may include multiple individual sensors, such as a gyro sensor, an acceleration sensor, or a composite sensor including two or more sensor fusions. A posture of the user, whether the user lies down, sits, or stands, may be measured by transmitting sensing result data of the activity sensor to the processor (103 of FIG. 1). According to one embodiment, the measured posture may be considered together with the sleep of the user measured through the EEG and/or the sleep level.

### Example of rear view of the earbud device 100

Referring to FIG. 2B, at least one ear wing electrode 210, 220 may be disposed on a side surface that is in contact with the inside of the antihelix of the user in the portion 142 of the ear wing 140. The electrode 210, 220 may be a signal electrode for the EEG measurement unit 150 to measure an EEG. The electrode 210, 220 may be a conductive metal material but may be another known material that minimizes the formation of a conductive polymer surface oxide film. While the elastic structure 141 condenses and restores, in an aspect that the electrode 210, 220 is pushed strongly toward the inner wall of the antihelix of the user and a contact force is maintained and in an aspect that an electrical connection is maintained even if the user moves during sleeping, the continuity of EEG measurement may be secured and since the inner wall of the antihelix may be an excellent measurement part similarly to the inner wall of the ear canal of the ear in an aspect of contact resistance, the structure of the device 100 may be designed to be advantageous to biometric information measurement.

A kernel 131 of the ear tip 130 may function as a sound output unit of an in-ear type earphone and when the device 100 performs sleep monitoring and user guide, the kernel 131 may output an appropriate notification sound and a voice information sound. The ear tip 130 may be conductive rubber or other conductive polymer materials and may be a flexible conductor and in this case, a surface layer of the ear tip 130 may be an ear tip electrode. However, the embodiment is not limited to one specific embodiment, and other embodiments may be possible.

The surface skin of the ear tip 130 may be a flexible electrode, such as conductive rubber or conductive polymer, as described above, but the ear tip electrode (not shown) may be separately provided. When the ear tip electrode is separately provided, portions other than the electrode may be an insulating material, such as insulating silicone, insulating rubber, or insulating polymer. In addition, a charging terminal 240 used for charging a battery of the earbud device 100 is illustrated.

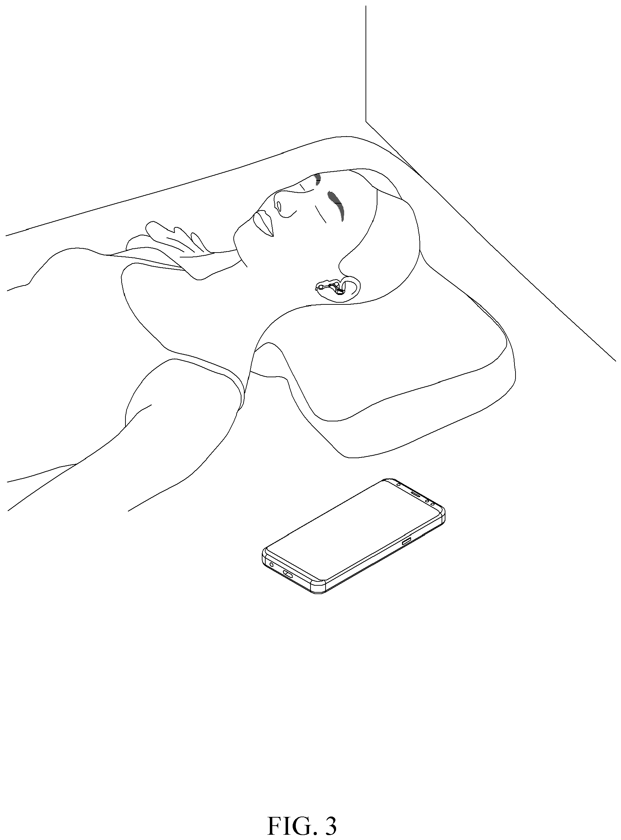

FIG. 3 is a diagram illustrating a process of sleep monitoring while wearing the earbud device 100 and sleeping according to one embodiment.

The earbud device 100 may be provided as a type worn on a left ear and a type worn on a right ear and typically, the user may select one from them and may purchase and wear it. The blood oxygen saturation measurement unit as shown in the drawings may be closely in contact with the earlobe of the user and may be fixed thereto; the ear tip may be inserted into the neck of the ear canal of the user, and the ear wing may be engaged with the inside of the antihelix and fixed, and the user may go to bed. The ear tip electrode and the ear wing electrode may function as a signal electrode and may maintain a stable electrical contact during sleep by the structure described above, as shown in the drawings, stable sleep monitoring may be possible while the user lies down and sleeps. As described with reference to FIG. 1, the sleep monitoring device 101 may be implemented in a smartphone connected via communication around the user.

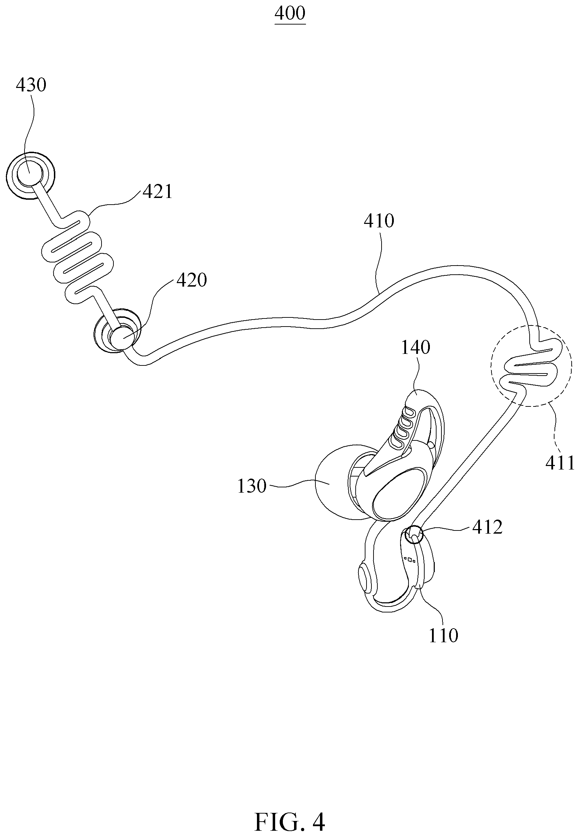

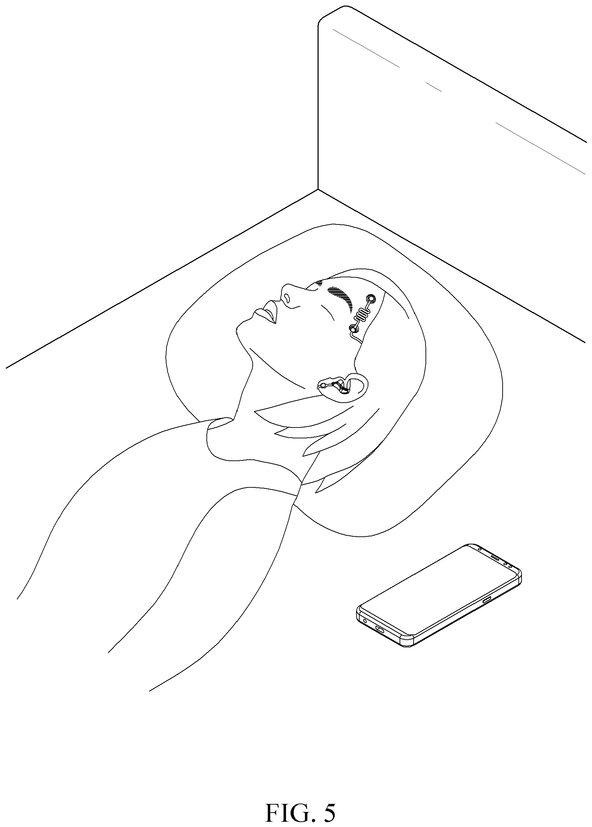

FIG. 4 is a diagram illustrating an earbud device 400 including a forehead expansion electrode according to another embodiment and FIG. 5 illustrates a user wearing the device and sleep monitoring is performed.

### Example of configuration and front view of the expanded earbud device 400

Since the configuration of the basic earbud device is described with reference to FIGS. 2A and 2B, hereinafter, a difference of the expanded earbud 400 is mainly described. The difference from the basic earbud device 100 may be the presence of expansion electrodes 420 and 430, and a connection line 410 for electrical connection to the expansion electrodes. The connection line 410 may be connected to an EEG measurement unit through a connector 412 of the first part 110 of the blood oxygen saturation measurement unit. The connector 412 may be a connector providing electrical connection, and various shapes and engagement structures may be possible. The expansion electrodes 420 and 430 may be electrodes respectively attached to the temple and forehead of the user. The electrode 420 may be attached to the temple in the form of patch electrode and the electrode 430 may be attached to the forehead in the form of patch electrode. The expansion electrodes 420 and 430 may be a disposable product or a consumable product assumed to be replaced after several uses.

Meanwhile, the connection line 410 may include folding structures 411 and 421 to adaptively correspond to the size and shape of the person's head. By the folding structure, the expanded earbud 400 may respond to persons with large heads and small heads as the folding structure is partially unfolded, and thereby, comfort may be provided to the person who wears the expanded earbud 400, and a function for the expansion electrodes 420 and 430 to be attached well without being removed as the expansion electrodes are pulled during the sleep may be provided. FIG. 5 illustrates a view in which a user wears an electronic device having an earbud expansion electrode and sleep monitoring is performed according to the embodiment of FIG. 4.

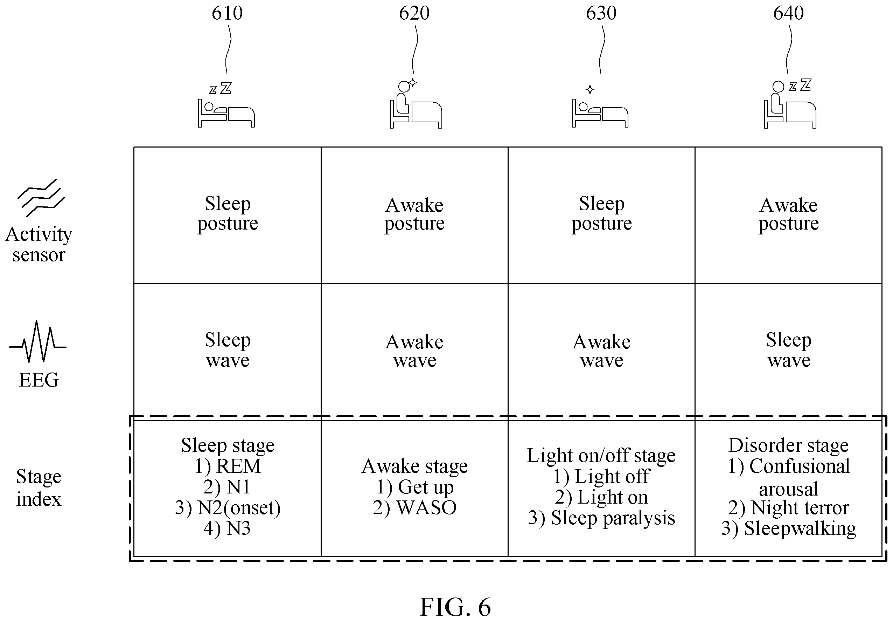

FIG. 6 illustrates a process of classifying a sleep state according to one embodiment and FIG. 7 illustrates a reason an earbud-type device has an advantage over a conventional watch-type device for sleep monitoring.

### Categorizing a sleep state by considering an activity sensor with EEG and advantages thereof

Referring to a table shown in FIG. 6, the states described with reference to FIG. 1 may be understood.

The first row of the table may be a result of classifying the user's posture as a lie-down posture (or a sleeping posture) and an activity posture (or an awake posture) by the processor 103 that receives the data of the activity sensor. The second row of the table may be a result of classifying a brain wave state of the user as a sleep wave and an awake wave by the processor 103 that receives the data measured by the EEG measurement unit. A total of four sleep states may be suggested by combining two posture types and two brain wave types.
1) lie-down posture and sleep wave state (SS) 610;
2) activity posture and awake wave state (AA) 620;
3) lie-down posture and awake wave state (SA) 630; and
4) activity posture and sleep wave state (AS) 640

The four states are described above. As described above, the posture of the user may be considered together with the brain wave (EEG) of the user and since the earbud device 100 is inserted into the user's ear, the posture may be able to accurately distinguish the user's state by comparing whether the user actually lies down and falls asleep compared to checking the sleep state by a conventional watch-type wearable device.

Referring to the example shown in FIG. 7, in two usage cases 710 and 720, a user's sleep may be determined when the earbud device 100 according to one embodiment of the present disclosure. Since the earbud device 100 is worn on the user's ear and the activity sensor in the earbud device estimates the user's posture, the sleep monitoring device 101 may accurately determine that the user lies down through a sensing value even if the user lies down on their back or on their side. However, in the two figures shown below, since the user's posture is estimated by a wearable device worn on the wrist as a watch-type, a posture of the wrist in a lie-down state of the user (lower left) may be the same as a posture of the wrist in a state in which the user raises their body and sits (lower right), the user's posture may not be accurately determined. As described above, estimation of the user's posture through the earbud device 100 worn on the ear according to the embodiments of the present disclosure may greatly help accurate estimation of the user's posture during sleep monitoring. In addition, the earbud device 100 may be optimized for sleep monitoring in various aspects, such as wearability, stability, and the possibility of maintaining electrical contact, compared to other types, such as hanging on the ear in the form of hook or covering the ear.

### Example of visualization of sleep monitoring result

FIG. 8 illustrates an example of a result screen in which sleep states are time sequentially generated and visualized according to one embodiment. As shown in the drawings, but without being limited thereto, the processor 103 may continuously classify a user's sleep state as one of SS, AA, SA, and AS during a time interval from 10:15 corresponding to an entry to sleep of the user to 06:50 corresponding to an exit of sleeping. In addition, the result thereof may be visualized and expressed in the flow of time as shown in the drawings. In addition, as described above, an interval is expressed as five minutes for ease of description and drawings, the length of an individual time interval may be set to be a different length, such as 30 seconds or one minute.

In the illustrated hypothetical situation, a five-minute interval around 10: 10 may be a last interval in which the user raises their head and since the user lays their head down at 10:15 and stays for more than a predetermined time (for example, more than 10 minutes), a sleep state may be monitored from 10:15 to a time point of exiting sleep (approximately 06:45) when the user wakes up from sleep and raises their body.

As described above, a first "SS" stage at 10:25, which is a first change from the "SA" state to the "SS" state in which the user lies down but does not fall asleep and then finally falls asleep may be a REM sleep level 2 stage, which is not REM sleep, among sleep levels determined by a sleep monitoring device 101 by analyzing an EEG signal of an earbud device 100. However, various other "SS" stages thereafter may include all of REM sleep and non-REM level 1 sleep.

Since the user tries to fall asleep for 10 minutes (two intervals from 10:15 to 10:25) while lying down and sleeps, a process of entering sleep may be relatively smooth without a sleep disorder. However, (if an outlier is properly removed from the result) a time interval of 02:10 to 02:15 may be classified as AS that the user raises their body more than a predetermined level. A sleep disorder may need to be checked because the user raises their body while the user is not awake, and if the event is repeated, an additional method, such as an imaging examination, may need to be considered depending on a pattern or a duration thereof.

An interval between 05:35 to 05:40 may be classified as AA in which the user wakes up from sleep and raises their body and it seems that the user lays their body down and tries to sleep (SA) and falls asleep, and the user may know, it may be a case in which the user wakes up for a moment in the early morning and goes to the bathroom. If the event also frequently repeats every day, the user may need to change a lifestyle habit, such as not drinking water for a couple of hours before sleeping or may need to be treated by a urologist, as necessary.

In the illustrated hypothetical example, the user wakes up around 06:40, lies down for about five minutes, and then raises their body and becomes active after 06:45. Accordingly, it may be determined that a sleep result of entering and exiting sleep for the user is moderate without an unusual item.

Other than the example shown in FIG. 8, various other visualizations, such as a clock type, a bar chart type, and a pie chart type, may be possible and this may be variously modified with ordinary knowledge in the infographics field.

### Example of sleep quality assessment result

FIG. 9 is an example of a screen providing an example of sleep quality and sleep disorder in a sleep monitoring result according to one embodiment and the screen may be output on the sleep monitoring device 101 or other displays connected thereto.

In the illustrated example, after the sleep monitoring process is terminated, a sleep monitoring device 9101 may perform a sleep quality assessment for the total monitored time interval (for example, 7 hours from entering sleep to exiting sleep).

According to one embodiment, the processor may provide a sleep quality index corresponding to a ratio of a sum of intervals classified as the lie-down posture and sleep wave state (SS) to a total time interval, based on the classification result. For example, but without being limited thereto, when a sum of time lengths of the lie-down posture and sleep wave (SS) state is 5 hours 15 minutes, to a total length of sleep time, the overall sleep quality may be assessed as 75 points. Since a state in which the user lies down to sleep and then raises their body (if the user is awake, the state is AA and if not, the state is AS) and a state in which the user lies down but is awake (SA) are not good for the overall sleep efficiency, when such states do not appear or are short, the sleep quality index may increase.

As shown in the lower portion of the left screen of FIG. 9, detailed sleep levels (REM, level 1, level 2, level 3, etc.) may be expressed or various other visual assessments may be possible.

In addition, as shown in the right screen of FIG. 9, a visualized result may be provided, such as providing sleep apnea, a snoring interval, and a change in a heart rate during sleeping. Preferably, more advanced diagnosis and analysis may be provided through an artificial intelligence server accessed by a network or a pre-designed algorithm, an unusual item or a suspected disease may be suggested, and a contact with an expert, such as a doctor, may be recommended if necessary.

### Estimating and analyzing sleep disorder or physical disease (suspected) states other than sleep quality

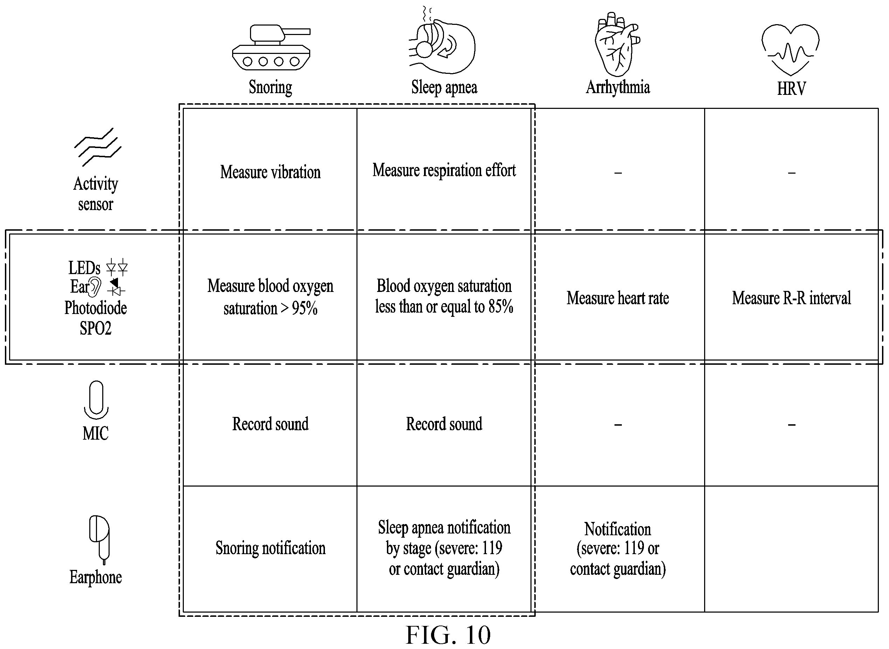

FIG. 10 is a diagram illustrating a sensor used by the sleep monitoring device 101 to monitor various sleep disorder events together with the earbud device 100 other than described above.

To identify snoring, the activity sensor may measure vibration, an oxygen saturation sensor may measure oxygen saturation, and a microphone may record snoring in an interval with snoring. As described above, the sleep monitoring device 101 may provide required guidance or notification to the user through the earbud device 100 as well as sleep monitoring, and in the case of severe snoring, the sleep monitoring device 101 may stimulate the user by providing a constant sound to the user to perceive. The advantage of this may be that, when the snoring notification is performed through a smartphone app, another person sleeping next to the user who snores may be disturbed by the notification, but according to the embodiment, only the person wearing the earbud device 100 on their ear may hear the notification and may be improved with an effort to change a sleep state for a moment.

In addition, as described above, since a motion or head vibration of the user is measured through the activity sensor, a snoring sound input through the microphone may be determined whether the sound is the snoring of the user or the snoring of the other person sleeping next to the user. For example, but without being limited thereto, when a vibration at a predetermined level is sensed together with the snoring sound, the sound may be the snoring of the user and when the snoring sound is collected but the vibration is less than or equal to the predetermined level, the sound may be the snoring of the person sleeping next to the user, and thereby, this may be ignored in the sleep state assessment of the user, who wears the earbud device and sleeps, subjected to monitoring. The sleep apnea is the same as the drawings.

Furthermore, since arrhythmias or other cardiac malfunctions may be monitored by measuring oxygen saturation, the utility of the earbud device 100 and the sleep monitoring device 101 may be great according to embodiments.

The units described herein may be implemented using a hardware component, a software component and/or a combination thereof. A processing device may be implemented using one or more general-purpose or special purpose computers, such as, for example, a processor, a controller and an arithmetic logic unit (ALU), a DSP, a microcomputer, an FPGA, a programmable logic unit (PLU), a microprocessor or any other device capable of responding to and executing instructions in a defined manner. The processing device may run an operating system (OS) and one or more software applications that run on the OS. The processing device also may access, store, manipulate, process, and create data in response to execution of the software. For purpose of simplicity, the description of a processing device is used as singular; however, one skilled in the art will appreciate that a processing device may include multiple processing elements and multiple types of processing elements. For example, the processing device may include a plurality of processors, or a single processor and a single controller. In addition, different processing configurations are possible, such as parallel processors.

The software may include a computer program, a piece of code, an instruction, or some combination thereof, to independently or uniformly instruct or configure the processing device to operate as desired. Software and data may be embodied permanently or temporarily in any type of machine, component, physical or pseudo equipment, computer storage medium or device, or in a propagated signal wave capable of providing instructions or data to or being interpreted by the processing device. The software also may be distributed over network-coupled computer systems so that the software is stored and executed in a distributed fashion. The software and data may be stored by one or more non-transitory computer-readable recording mediums.

The methods according to the above-described example embodiments may be recorded in non-transitory computer-readable media including program instructions to implement various operations of the above-described example embodiments. The media may also include, alone or in combination with the program instructions, data files, data structures, and the like. The program instructions recorded on the media may be those specially designed and constructed for the purposes of example embodiments, or they may be of the kind well-known and available to those having skill in the computer software arts. Examples of non-transitory computer-readable media include magnetic media such as hard disks, floppy disks, and magnetic tape, optical media such as CD-ROM discs, DVDs, and/or Blue-ray discs, magneto-optical media such as optical discs, and hardware devices that are specially configured to store and perform program instructions, such as read-only memory (ROM), random access memory (RAM), flash memory (e.g., USB flash drives, memory cards, memory sticks, etc.), and the like. Examples of program instructions include both machine code, such as produced by a compiler, and files containing higher-level code that may be executed by the computer using an interpreter. The above-described devices may be configured to act as one or more software modules in order to perform the operations of the above-described example embodiments, or vice versa.

A number of example embodiments have been described above. Nevertheless, it should be understood that various modifications may be made to these example embodiments. For example, suitable results may be achieved if the described techniques are performed in a different order and/or if components in a described system, architecture, device, or circuit are combined in a different manner and/or replaced or supplemented by other components or their equivalents.

Therefore, other implementations, other examples, and equivalents to the claims are also within the scope of the following claims.

## Claims

1. A user sleep monitoring device, the sleep monitoring device comprising:
a communication interface; and
a processor,
wherein the processor is configured to determine whether a brain wave of the user corresponds to a sleep wave or an awake wave using an electroencephalogram (EEG) signal measured by an EEG measurement unit of an earbud device worn on an ear of the user, and classify a sleep state of the user as one of a plurality of categories by comparing the determination result with sensor data measured by an activity sensor comprised in the earbud device, wherein the sensor data is time synchronized to the EEG signal.

2. The sleep monitoring device of claim 1,
wherein the processor is configured to determine whether the sensor data measured by the activity sensor corresponds to an activity posture or a lie-down posture, and classify the sleep state of the user as one of a plurality of states comprising:
1) lie-down posture and sleep wave state (SS);
2) activity posture and awake wave state (AA);
3) lie-down posture and awake wave state (SA); and
4) activity posture and sleep wave state (AS).

3. The sleep monitoring device of claim 2,
wherein the processor is configured to continuously classify the sleep state of the user as one of the plurality of states during a time interval between a first time point corresponding to an entry to sleep of the user and a second time point corresponding to exiting sleep of the user and provide a result sequence of the state classification over time as a sleep monitoring result of the user.

4. The sleep monitoring device of claim 3,
wherein the processor is configured to classify the sleep state of the user as one of the plurality of states at a predetermined cycle during the time interval.

5. The sleep monitoring device of claim 3,
wherein the processor is configured to, when the sleep state of the user is classified as one of the plurality of states at the predetermined cycle, apply a smoothing filter to remove an outlier from at least one piece of the sensor data measured by the activity sensor and the EEG signal within a cycle and determine the sleep state of the user corresponding to the cycle to be one of the plurality of states.

6. The sleep monitoring device of claim 3,
wherein the processor is configured to, when the sleep state of the user is classified as one of the plurality of states at the predetermined cycle, segment one cycle into a plurality of time segments, determine the sleep state of the user corresponding to each time segment as one of the plurality of states using the sensor data measured by the activity sensor and the EEG signal for each of the plurality of time segments, and determine a major state that forms the majority for the cycle to be a representative state of the cycle.

7. The sleep monitoring device of claim 3,
wherein the processor is configured to provide a sleep quality index corresponding to a ratio of a sum of intervals classified as the lie-down posture and sleep wave state (SS) to an entirety of the time intervals, based on the classification result.

8. The sleep monitoring device of claim 2,
wherein the processor is configured to, when classifying:
after the activity posture and awake wave state (AA) changes to the lie-down posture and awake wave state (SA), in case of a first change from the lie-down posture and awake wave state (SA) to the lie-down posture and sleep wave state (SS), when the EEG signal is a non-rapid eye movement (REM) level 2 sleep state, classify the sleep state of the user as the lie-down posture and sleep wave state (SS).

9. The sleep monitoring device of claim 1,
wherein the sensor data measured by the activity sensor comprises:
a direction sensing value that determines whether the user raises or lays down their head while wearing the earbud device on the ear; and
an acceleration sensing value that determines whether the user moves at a predetermined level or more while wearing the earbud device on the ear, and
wherein the processor is configured to temporarily defer the classification when the acceleration sensing value indicates the state in which the user moves at the predetermined level or more.

10. The sleep monitoring device of claim 9,
wherein the sleep monitoring device is configured to further receive a sound collected through a microphone of the earbud device and when the sound corresponds to snoring, distinguish whether the sound is snoring of the user or snoring of other surrounding person using the acceleration sensing value.
